# EUROPÄISCHE PATENTSCHRIFT

(11) **EP 3 752 480 B1**
(45) Veröffentlichungstag und Bekanntmachung des Hinweises auf die Patenterteilung: **21.06.2023**
(21) Anmeldenummer: 19703099.2
(22) Anmeldetag: 06.02.2019
(51) Int. Cl.: C09K 23/36

(54) **VERFAHREN ZUR HERSTELLUNG EINER OBERFL& XC4;CHENAKTIVEN MISCHUNG UMFASSEND KONDENSATIONSPRODUKTE VON & X391;-HYDROXYCARBONS& XC4;UREN MIT 1,2-ALKANDIOLEN**
METHOD FOR PRODUCING A SURFACE-ACTIVE MIXTURE COMPRISING CONDENSATION PRODUCTS OF ALPHA-HYDROXY CARBOXYLIC ACIDS WITH 1,2-ALKANE DIOLS
PROCÉDÉ POUR PRODUCTION D'UN MÉLANGE TENSIOACTIF COMPORTANT DES PRODUITS DE CONDENSATION D'ACIDES ALPHA-HYDROXYCARBOXYLIQUES AYANT 1,2-ALCANEDIOLS

(30) Priorität: 14.02.2018 EP 18156754
(43) Veröffentlichungstag der Anmeldung: 23.12.2020
(73) Patentinhaber: BASF SE, 67056 Ludwigshafen am Rhein (DE); Bergische Universität Wuppertal, 42119 Wuppertal (DE)
(72) Erfinder: WEICHOLD, Catherine, 40589 Düsseldorf-Holthausen (DE); BEHLER, Ansgar, 40589 Düsseldorf-Holthausen (DE); MELCHIOR, David, 40789 Monheim (DE); BUSCH, Stefan, 40589 Düsseldorf-Holthausen (DE); KLING, Hans-Willi, 42119 Wuppertal (DE); LANGE, Karsten, 42119 Wuppertal (DE); JAKOB, Bernd, 42119 Wuppertal (DE)
(74) Vertreter: BASF IP Association
(86) Internationale Anmeldenummer: PCT/EP2019/052848
(87) Internationale Veröffentlichungsnummer: WO 2019/158410

(56) Entgegenhaltungen:
- DE-A1-102011 054 602
- US-A- 3 102 128
- US-A- 5 089 658
- Anonymous: "FNR: Projektverzeichnis - Details", , 14. Januar 2016 (2016-01-14), XP055496892, Gefunden im Internet: URL:https://www.fnr.de/index.php?id=11150& fkz=22041911 [gefunden am 2018-08-02]
- Anonymous: "FNR: Projektverzeichnis - Details", , 30. April 2016 (2016-04-30), XP055496884, Gefunden im Internet: URL:https://www.fnr.de/index.php?id=11150& fkz=22042011 [gefunden am 2018-08-02]

## Beschreibung

Die vorliegende Erfindung betrifft ein Verfahren zur Herstellung einer oberflächenaktiven Mischung umfassend Kondensationsprodukte, wobei eine α-Hydroxycarbonsäure mit 3 bis 6 Kohlenstoffatomen mit mindestens einem 1 ,2-Alkandiol umgesetzt wird. Weiterhin betrifft die Erfindung eine nach dem erfindungsgemäßen Verfahren herstellbare oder hergestellte oberflächenaktive Mischung. Die Erfindung betrifft ebenfalls die Verwendung der nach dem erfindungsgemäßen Verfahren herstellbaren oder hergestellten Mischung als Tensid und/oder Emulgator, vorzugsweise in einem Wasch- oder Reinigungsmittel und/oder in einem Kosmetikprodukt. Die Erfindung betrifft auch ein Wasch- oder Reinigungsmittel und/oder ein Kosmetikprodukt, welches jeweils eine nach dem erfindungsgemäßen Verfahren herstellbare oder hergestellte Mischung enthält.

Es sind bereits verschiedene, auch oberflächenaktive, Verbindungen bekannt, welche durch Umsetzung einer α-Hydroxycarbonsäure wie Citronensäure mit ein- oder mehrwertigen Alkoholen erhalten werden können.

So wird z.B. im Dokument WO 2013/057110A1 (entspricht DE 102011054602 A1) ein Borkatalysiertes Verfahren zur gezielten Synthese asymmetrischer Monoester und/oder symmetrischer Diester der Citronensäure mit ein- oder mehrwertigen Alkoholen beschrieben.

Im Dokument WO 2010/112459 werden Zusammensetzungen zur Körperpflege beschrieben, enthaltend einen schweißhemmenden Wirkstoff, Wasser, einen Ester - ausgewählt aus den Einfach- und Mehrfachestem von Milchsäure, Citronensäure, Weinsäure oder Adipinsäure mit einem zwei-, drei- oder vierwertigen Alkohol mit 2 bis 9 Kohlenstoffatomen - und ein unter Normalbedingungen flüssiges kosmetisches Öl.

In dem Dokument US 5,089,658 A werden Citronensäureester als u.a. Reaktivverdünner für wärmehärtbare Beschichtungen angegeben.

Das Dokument US 3,102,128 A beschreibt gemischte Ester und ein Verfahren zu deren Herstellung.

In dem Dokument mit der Bezeichnung "Oberflächenaktive Verbindungen auf Basis von Polymerkondensaten aus Fettderivaten, Hydroxycarbonsäuren und Polyolen" der Fachagentur für Nachwachsende Rohstoffe e.V. (zugänglich unter der Internet-Adresse: https://www.fnr.de/index.php?id=11150&fkz=22042011) wird über ein Verfahren zur Herstellung oberflächenaktiver, fettalkoholmodifizierter Citronensäuredimere berichtet.

In dem Dokument mit der Bezeichnung "Oberflächenaktive Kombinationsprodukte aus epoxidierten Fettderivaten" der Fachagentur für Nachwachsende Rohstoffe e.V. (zugänglich unter der Internet-Adresse: https://www.fnr.de/index.php?id=11150&fkz=22041911) wird über ein Verfahren zur Herstellung von oberflächenaktiven Kombinationsprodukten aus epoxidierten Fettderivaten und Hydroxycarbonsäuren berichtet, welche als Tenside oder Emulgatoren geeignet sind.

Es besteht aber auch angesichts des bekannten Standes der Technik weiterhin ein Bedürfnis nach einem einfachen, wirtschaftlichen und umweltverträglichen Verfahren um oberflächenaktive Verbindungen, insbesondere Tenside und/oder Emulgatoren herzustellen. Ebenso besteht ein Bedürfnis, Wasch- bzw. Reinigungsmittel und/oder Kosmetikprodukte nach einem solchen Verfahren herzustellen, welche möglichst wenige und vorzugsweise keine Anteile von organischen Lösungsmitteln und/oder von Katalysatoren wie Bor-haltigen Katalysatoren enthalten. "Umweltverträglich" im Sinne der vorliegenden Erfindung bedeutet vorzugsweise, dass ein Verfahren mit dieser Eigenschaft unter überwiegendem Einsatz nachwachsender Rohstoffe sowie mit einem möglichst geringen Anfall von für den vorgesehenen Verwendungszweck unbrauchbaren Nebenprodukten (einschließlich Lösungsmittel), vorzugsweise ohne Anfall an für den vorgesehenen Verwendungszweck unbrauchbaren Nebenprodukten, durchgeführt wird bzw. werden kann. Als nachwachsende Rohstoffe gelten dabei im Rahmen der vorliegenden Erfindung auch aus nachwachsenden Rohstoffen, insbesondere aus Pflanzen, durch nachfolgende Umsetzungs- bzw. Veredelungsschritte gewonnene Rohstoffe, sowie Rohstoffe, die durch Mikroorganismen hergestellt werden können.

Es war daher eine primäre Aufgabe der vorliegenden Erfindung, ein Verfahren zur Herstellung eines oberflächenaktiven Produktes bereitzustellen, vorzugsweise ein einfaches, wirtschaftliches Verfahren, welches mit geringem Aufwand die Herstellung von Tensiden und/oder Emulgatoren ermöglicht, welche zum Einsatz in Wasch- bzw. Reinigungsmitteln und/oder in Kosmetikprodukten geeignet sind. Ein geringer Aufwand sollte vorzugsweise dadurch erreicht werden, dass das Verfahren keinen Wechsel des Reaktionsgefäßes ("Eintopfreaktion") und/oder eines Lösungsmittels erfordert und/oder dadurch, dass keine aufwändigen Reinigungs- oder Isolierungsschritte zur Gewinnung bzw. Isolierung des Verfahrensproduktes nötig sind.

Eine weitere Aufgabe der vorliegenden Erfindung war es, ein vorgenanntes Verfahren bereitzustellen, welches weitgehend umweltverträglich ist und vorzugsweise weder den Einsatz von Lösungsmitteln noch von Katalysatoren, beispielsweise Borsäure oder Boronsäuren, erfordert, so dass die resultierenden Verfahrensprodukte möglichst wenige und vorzugsweise keine Anteile von organischen Lösungsmitteln und/oder von Katalysatoren wie Bor-haltigen Katalysatoren enthalten.

Eine weitere spezifische Aufgabe der vorliegenden Erfindung bestand darin, ein Wasch- oder Reinigungsmittel und/oder ein Kosmetikprodukt zur Verfügung zu stellen, welches möglichst umweltverträglich, einfach und kostengünstig hergestellt werden kann und welches möglichst wenige und vorzugsweise keine Anteile von organischen Lösungsmitteln und/oder von Katalysatoren wie Bor-haltigen Katalysatoren enthält.

Die Erfindung sowie erfindungsgemäß bevorzugte Kombinationen bevorzugter Parameter, Eigenschaften und/oder Bestandteile der vorliegenden Erfindung werden in den beigefügten Ansprüchen definiert. Bevorzugte Aspekte der vorliegenden Erfindung werden auch in der nachfolgenden Beschreibung sowie in den Beispielen angegeben bzw. definiert.

Sofern im Folgenden Verfahren zur Herstellung einer oberflächenaktiven Mischung umfassend Kondensationsprodukte, erfindungsgemäße oberflächenaktive Mischungen umfassend Kondensationsprodukte, erfindungsgemäße Verwendungen von erfindungsgemäßen oberflächenaktiven Mischung umfassend Kondensationsprodukte und/oder erfindungsgemäße Wasch- bzw. Reinigungsmittel oder Kosmetikprodukte beschrieben werden, welche näher bestimmte Ausführungsformen, Bestandteile, Verbindungen oder Merkmale "umfassen" oder "enthalten", soll jeweils auch die in einem engeren Umfang zu verstehende entsprechende Definition mit offenbart sein, worin die besagten Verfahren zur Herstellung einer oberflächenaktiven Mischung umfassend Kondensationsprodukte, die erfindungsgemäßen oberflächenaktiven Mischungen umfassend Kondensationsprodukte, die Verwendungen von erfindungsgemäßen oberflächenaktiven Mischung umfassend Kondensationsprodukte und/oder erfindungsgemäßen Wasch- bzw. Reinigungsmittel oder Kosmetikprodukte aus diesen jeweils näher bestimmten Ausführungsformen, Verbindungen, Bestandteilen oder Merkmalen "bestehen".

Es wurde nun gefunden, dass die primäre Aufgabe sowie weitere Aufgaben und/oder Teilaufgaben der vorliegenden Erfindung gelöst werden durch ein erfindungsgemäßes Verfahren gemäß Anspruch 1 der vorliegenden Schrift, wobei die in diesem Anspruch genannte Temperatur vorzugsweise im Bereich von 115 bis 140 °C, besonders bevorzugt im Bereich von 120 bis 135 °C liegt.

In dem erfindungsgemäßen Verfahren ist die α-Hydroxycarbonsäure mit 3 bis 6 Kohlenstoffatomen vorzugsweise ausgewählt aus der Gruppe bestehend aus Äpfelsäure, Citronensäure, Isocitronensäure, Weinsäure und deren Gemischen, besonders bevorzugt aus der Gruppe bestehend aus Äpfelsäure, Citronensäure und deren Gemischen, wobei ganz besonders bevorzugt die α-Hydroxycarbonsäure Citronensäure ist. Sofern eine der vorgenannten α-Hydroxycarbonsäuren in stereoisomeren Formen auftritt, sind im Rahmen der vorliegenden Erfindung alle diese Stereoisomere und auch deren Mischungen in beliebigen Mischungsverhältnissen, insbesondere deren Racemate, von den vorstehend angegebenen Definitionen umfasst, insbesondere D-Äpfelsäure, L-Äpfelsäure, racemische D,L-Äpfelsäure, D-Weinsäure, L-Weinsäure, racemische D,L-Weinsäure, meso-Weinsäure und L-threo-Isocitronensäure.

In eigenen Versuchen wurde festgestellt, dass bei einer Umsetzung nach dem erfindungsgemäßen Verfahren im bevorzugten Temperaturbereich von 115 bis 140 °C, besonders bevorzugt im Temperaturbereich von 120 bis 135 °C und ganz besonders bevorzugt bei 130 °C, die besten Ergebnisse hinsichtlich Vollständigkeit des Reaktionsumsatzes, Homogenität der Reaktionsmischung sowie Wasserlöslichkeit der entstandenen oberflächenaktiven Mischung umfassend Kondensationsprodukte erzielt werden. Eine höhere Wasserlöslichkeit der entstandenen oberflächenaktiven Mischung umfassend Kondensationsprodukte wurde unter diesen Bedingungen insbesondere festgestellt, sofern n-1,2-Decandiol oder n-1,2-Dodecandiol als 1,2-Alkandiole der Formel I eingesetzt wurden. Weiterhin wurden die genannten Vorteile insbesondere festgestellt, sofern als α-Hydroxycarbonsäure Citronensäure und/oder Äpfelsäure, vorzugsweise Citronensäure, eingesetzt wurde. Höhere Reaktionstemperaturen sind auch möglich, vorzugsweise bei entsprechender Anpassung (Verkürzung) der Reaktionszeiten, aber wegen des höheren Energieaufwandes zumindest unwirtschaftlich. Bei Reaktionstemperaturen > 150 °C können außerdem leicht Zersetzungsprozesse, beispielsweise der Edukte, auftreten; solche Reaktionstemperaturen > 150 °C sind daher aus den vorgenannten Gründen nicht bevorzugt.

Vorzugsweise bedeutet die angegebene Temperatur im Bereich von 110 bis 150 °C bzw. in den entsprechenden bevorzugten Bereichen hier und im Folgenden jeweils die Temperatur der (äußeren) Heizvorrichtung, beispielsweise des Heizbades wie eines Ölbades.

Vorzugsweise bedeutet in dem erfindungsgemäßen Verfahren die Anwesenheit von Wasser hier und im Folgenden, dass Wasser in einem molaren Mengenverhältnis im Bereich von 0,5 : 1 bis 20 : 1, vorzugsweise im Bereich von 1 : 1 bis 10 : 1, bezogen auf die eingesetzte molare Menge an α-Hydroxycarbonsäure (bzw. an Gemisch an α-Hydroxycarbonsäuren) eingesetzt wird.

Bevorzugt ist ein erfindungsgemäßes Verfahren,
wobei
die Umsetzung zumindest zeitweilig in einem homogenen Reaktionsgemisch durchgeführt wird
   und/oder
das homogene Reaktionsgemisch erzeugt wird, indem
   (i) die α-Hydroxycarbonsäure in Wasser gelöst wird
      und anschließend
   (ii) das 1,2-Alkandiol der Formel I zu der resultierenden Lösung hinzugegeben und das resultierende Reaktionsgemisch bei der Reaktionstemperatur im Bereich von 110 bis 150 °C, vorzugsweise im Bereich von 115 bis 140 °C, besonders bevorzugt im Bereich von 120 bis 135 °C und insbesondere bevorzugt bei 130 °C, mechanisch durchmischt wird.

Die Umsetzung wird in der vorstehend angegebenen bevorzugten erfindungsgemäßen Verfahrensvariante vorzugsweise für den überwiegenden Teil der gesamten Reaktionsdauer in einem homogenen Reaktionsgemisch durchgeführt, besonders bevorzugt für mindestens die Hälfte oder mehr der gesamten Reaktionsdauer. Homogen bedeutet in diesem Zusammenhang vorzugsweise einphasig flüssig.

Die α-Hydroxycarbonsäure (bzw. ein Gemisch an α-Hydroxycarbonsäuren) kann in dieser bevorzugten erfindungsgemäßen Verfahrensvariante sowie in anderen Verfahrensvarianten in einem Teil des insgesamt für die Umsetzung eingesetzten Wasservolumens gelöst werden oder vorzugsweise in dem vollständigen, insgesamt für die Umsetzung eingesetzten Wasservolumen. Vorzugsweise wird zum Lösen der α-Hydroxycarbonsäure(n) erwärmtes Wasser, vorzugsweise auf eine Temperatur im Bereich von 30 bis 90 °C erwärmtes Wasser, verwendet.

Das mechanische Durchmischen erfolgt in dieser bevorzugten erfindungsgemäßen Verfahrensvariante sowie in anderen Verfahrensvarianten vorzugsweise mittels eines geeigneten Rührers wie eines Ankerrührers oder eines Magnetrührers. Der Fachmann kann anhand der Konsistenz der Reaktionsmischung eine geeignete Methode zur mechanischen Durchmischung auswählen.

Bevorzugt ist weiterhin ein erfindungsgemäßes oder bevorzugtes erfindungsgemäßes Verfahren,
wobei
die α-Hydroxycarbonsäure ausgewählt ist aus der Gruppe bestehend aus Äpfelsäure, Citronensäure, Isocitronensäure, Weinsäure und deren Gemischen, vorzugsweise aus der Gruppe bestehend aus Äpfelsäure, Citronensäure und deren Gemischen, wobei besonders bevorzugt die α-Hydroxycarbonsäure Citronensäure ist;
und/oder
das molare Verhältnis des 1,2-Alkandiols zu der α-Hydroxycarbonsäure im Bereich von 1 : 1 bis 1: 2, vorzugsweise im Bereich von 1 : 1,5 bis 1: 2, liegt.

Vorzugsweise wird in dem erfindungsgemäßen oder einem bevorzugten erfindungsgemäßen Verfahren nur jeweils eine α-Hydroxycarbonsäure eingesetzt, d.h. reine α-Hydroxycarbonsäuren sind erfindungsgemäß gegenüber Gemischen von α-Hydroxycarbonsäuren bevorzugt.

In eigenen Versuchen wurde festgestellt, dass die Umsetzungen nach dem erfindungsgemäßen Verfahren zu den besten Ergebnissen führten, sofern als α-Hydroxycarbonsäure jeweils Äpfelsäure oder Citronensäure eingesetzt wurde. Weiter wurde in eigenen Versuchen festgestellt, dass vorzugsweise erwünschte wasserlösliche oberflächenaktive Mischungen umfassend Kondensationsprodukte, vorzugsweise mit vorteilhaften Eigenschaften als Tenside und/oder Schaumverbesserer und/oder Schaumverstärker, nach dem erfindungsgemäßen Verfahren erhalten wurden, sofern als α-Hydroxycarbonsäure Citronensäure eingesetzt wurde.

In eigenen Versuchen wurde außerdem festgestellt, dass besonders gute oberflächenaktive Eigenschaften der nach dem erfindungsgemäßen Verfahren hergestellten oberflächenaktiven Mischungen umfassend Kondensationsprodukte erzielt wurden (besonders gute Schaumeigenschaften von Tensiden, insbesondere besonders gute Schaumhöhen und/oder besonders gute Schaumqualitäten bzw. besonders gutes Emulgiervermögen und besonders gute Stabilität von Emulsionen, welche mit nach dem erfindungsgemäßen Verfahren hergestellten oberflächenaktiven Mischungen umfassend Kondensationsprodukte hergestellt wurden), sofern das molare Verhältnis des 1,2-Alkandiols zu der α-Hydroxycarbonsäure im bevorzugten Bereich von 1 : 1 bis 1: 2 bzw. im besonders bevorzugten Bereich von 1 : 1,5 bis 1: 2 lag.

In dem erfindungsgemäßen oder einem bevorzugten erfindungsgemäßen Verfahren wird mindestens ein 1,2-Alkandiol der Formel I wie vorstehend angegeben umgesetzt, d.h. es wird ein 1,2-Alkandiol der Formel I umgesetzt oder eine Mischung von zwei oder mehr 1,2-Alkandiolen der Formel I, wobei aber jeweils die Summe der molaren Mengen der zwei oder mehr 1,2-Alkandiole der Formel I ebenfalls in einem molaren (Gesamt-) Verhältnis zu der α-Hydroxycarbonsäure (bzw. zu dem Gemisch von α-Hydroxycarbonsäuren) im Bereich von 1 : 0,5 bis 1: 2,5 (bzw. den entsprechenden vorstehend angegebenen bevorzugten Bereichen) liegt. Vorzugsweise wird erfindungsgemäß nur ein 1,2-Alkandiol der Formel I wie vorstehend angegeben umgesetzt, d.h. reine 1,2-Alkandiole sind gegenüber Gemischen von 1,2-Alkandiolen bevorzugt

In einer bevorzugten Ausgestaltung des erfindungsgemäßen oder eines bevorzugten erfindungsgemäßen Verfahrens bedeutet R¹ in dem 1,2-Alkandiol der Formel I eine lineare (d.h. unverzweigte) Kohlenwasserstoffgruppe mit insgesamt 6 bis 18 Kohlenstoffatomen, vorzugsweise mit insgesamt 6 bis 16 Kohlenstoffatomen, besonders bevorzugt mit insgesamt 8 bis 14 Kohlenstoffatomen. In eigenen Versuchen wurde festgestellt, dass nach dieser Ausgestaltung hergestellte oberflächenaktive Mischungen umfassend Kondensationsprodukte besonders gute Eigenschaften als Tenside und/oder Schaumverbesserer und/oder Schaumverstärker und/oder Emulgatoren aufwiesen.

In einer bevorzugten spezifischen Ausgestaltung des erfindungsgemäßen oder eines bevorzugten erfindungsgemäßen Verfahrens bedeutet R¹ in dem 1,2-Alkandiol der Formel I eine lineare (unverzweigte) Kohlenwasserstoffgruppe mit insgesamt 6 bis 12 Kohlenstoffatomen, vorzugsweise mit insgesamt 8 bis 10 Kohlenstoffatomen, und besonders bevorzugt ist der oder mindestens ein 1,2-Alkandiol der Formel I n-1,2-Decandiol. In eigenen Versuchen wurde festgestellt, dass nach dieser spezifischen Ausgestaltung hergestellte oberflächenaktive Mischungen umfassend Kondensationsprodukte besonders gute Eigenschaften als Tenside und/oder Schaumverbesserer und/oder Schaumverstärker aufwiesen.

In einer weiteren bevorzugten spezifischen Ausgestaltung des erfindungsgemäßen oder eines bevorzugten erfindungsgemäßen Verfahrens bedeutet R¹ in dem 1,2-Alkandiol der Formel I eine lineare (d.h. unverzweigte) Kohlenwasserstoffgruppe mit insgesamt 14 bis 18 Kohlenstoffatomen, vorzugsweise mit insgesamt 14 bis 16 Kohlenstoffatomen, besonders bevorzugt mit insgesamt 14 Kohlenstoffatomen. In eigenen Versuchen wurde festgestellt, dass nach dieser spezifischen Ausgestaltung hergestellte oberflächenaktive Mischungen umfassend Kondensationsprodukte besonders gute Eigenschaften als Emulgatoren aufwiesen.

Bevorzugt ist ebenfalls ein erfindungsgemäßes oder bevorzugtes erfindungsgemäßes Verfahren,
wobei
die Umsetzung zumindest bis zum Erreichen einer konstanten "vorhandenen Säurezahl" und/oder eines konstanten pH-Wertes, vorzugsweise bis zum Erreichen einer konstanten "vorhandenen Säurezahl", durchgeführt wird,
und/oder
die hergestellte oberflächenaktive Mischung umfassend Kondensationsprodukte einen Anteil an nicht umgesetztem 1,2-Alkandiol der Formel I von bis zu 30 Gew.-%, vorzugsweise im Bereich von 10 bis 25 Gew.-%, umfasst, bezogen auf die Gesamtmasse (bzw. das Gesamtgewicht) des als Ausgangsverbindung eingesetzten1,2-Alkandiols der Formel I.

In der erfindungsgemäßen Verfahrensvariante, worin Wasser während der Umsetzung zumindest zeitweilig am Entweichen aus dem Reaktionsgefäß gehindert wird, wobei die Umsetzung vorzugsweise zumindest zeitweilig unter Rückflusskühlung durchgeführt wird ("geschlossenes System"), werden besonders gut reproduzierbare Ergebnisse erhalten hinsichtlich der oberflächenaktiven Eigenschaften der hergestellten oberflächenaktiven Mischungen umfassend Kondensationsprodukte, wie deren Tensideigenschaften (insbesondere erzielbare Schaumhöhe und/oder Schaumqualität) bzw. deren Emulgatoreigenschaften (insbesondere Stabilität der Emulsionen, welche mit nach dem bevorzugten erfindungsgemäßen Verfahren hergestellten Kondensationsprodukten hergestellt wurden).

In eigenen Untersuchungen wurde festgestellt, dass sich unter den vorgenannten Reaktionsbedingungen ("geschlossenes System") innerhalb einer Reaktionsdauer im Bereich von 3 bis 7 Stunden, vorzugsweise im Bereich von 4 bis 6 Stunden, ein Reaktionsgleichgewicht einstellte, bei dem vorteilhafte Produkteigenschaften erzielt wurden, die sich auch im weiteren zeitlichen Reaktionsverlauf nicht mehr nennenswert veränderten. Da demnach in dieser Verfahrensvariante eine laufende Kontrolle des Reaktionsfortschrittes nicht nötig erscheint, eignet sie sich besonders gut für eine Übertragung in größere Maßstäbe bis zum technischen Maßstab.

Demnach ist in vielen Fällen ein erfindungsgemäßes oder bevorzugtes erfindungsgemäßes Verfahren bevorzugt, wobei
Wasser während der Umsetzung zumindest zeitweilig, vorzugsweise andauernd über den gesamten Reaktionsverlauf, am Entweichen aus dem Reaktionsgefäß gehindert wird, wobei die Umsetzung vorzugsweise zumindest zeitweilig, besonders bevorzugt andauernd über den gesamten Reaktionsverlauf, unter Rückflusskühlung durchgeführt wird
und
die Reaktionsdauer im Bereich von 3 bis 7 Stunden, vorzugsweise im Bereich von 4 bis 6 Stunden, liegt.

Eine Kontrolle des Reaktionsfortschrittes nach dem erfindungsgemäßen Verfahren ist insbesondere im Fall des "geschlossenen Systems" (vor allem bei Umsetzungen von Citronensäure mit einem 1 ,2-Alkandiol der Formel I) möglich durch die Bestimmung der sogenannten "vorhandenen Säurezahl" ("vSZ"). Die vorhandene Säurezahl (in Einheiten von "mmol (OH⁻)/g Testsubstanz", welche zur Neutralisierung - Farbumschlag von Phenolphthalein von farblos nach rosa - pro g Testsubstanz erforderlich ist bzw. war; nähere Angaben zur Bestimmungsmethode siehe die nachfolgend angegebenen Beispiele) gibt an, wie viele Säureäquivalente sich in einem Gramm einer zu untersuchenden Testsubstanz (bzw. eines zu untersuchenden Testsubstanzgemisches) befinden (bzw. vor der Neutralisation befanden). Die Größe im Zähler der Bestimmungsgleichung für die vSZ gibt dabei an, welche Menge an Lauge zur Neutralisierung der in der Testsubstanz (bzw. dem Testsubstanzgemisch) enthaltenen Säureäquivalente verbraucht wird. Die Größe im Nenner der Bestimmungsgleichung für die vSZ gibt die Masse der Testsubstanz (bzw. des Testsubstanzgemisches) an, für welche die vorhandene Säurezahl ermittelt werden soll.

Der Begriff "konstante vorhandene Säurezahl" bedeutet im Rahmen der vorliegenden Erfindung, dass sich die in einer ersten Probe einer Umsetzung ermittelte vorhandene Säurezahl um nicht mehr als 5 % von den in zwei weiteren Proben derselben Umsetzung ermittelten vorhandenen Säurezahlen unterscheidet, welche im zeitlichen Abstand von einer Stunde bzw. von zwei Stunden nach der ersten Probe entnommen wurden (d.h. von den zwei weiteren Proben wird die erste weitere Probe im zeitlichen Abstand von einer Stunde nach der ersten Probe entnommen und die zweite weitere Probe wird im zeitlichen Abstand von zwei Stunden nach der ersten Probe entnommen).

Es wurde in eigenen Untersuchungen gefunden, dass in der Verfahrensvariante des "geschlossenen Systems" nach Einstellung eines wie vorstehend beschriebenen vorteilhaften Reaktionsgleichgewichtes die entsprechend hergestellte bzw. erhaltene oberflächenaktive Mischung umfassend Kondensationsprodukte eine konstante vorhandene Säurezahl aufwies, die in etwa dem theoretisch ermittelten Wert der vorhandenen Säurezahl für die jeweils eingesetzte, einfach veresterte α-Hydroxycarbonsäure (d.i. Monoester der eingesetzten α-Hydroxycarbonsäure mit dem eingesetzten 1 ,2-Alkandiol der Formel I, nachfolgend auch angegeben als "vSZ(Monoester)") entsprach (vgl. das unten in den Beispielen angegebene Berechnungsbeispiel für die Größe "vSZ(Monoester)" für ein "geschlossenes System").

Eine andere Variante des erfindungsgemäßen oder eines bevorzugten erfindungsgemäßen Verfahrens wird in einem "offenen System" (z.B. einem offenen Reaktionsgefäß) durchgeführt, d.h. die im Reaktionsgemisch enthaltenen oder gebildeten niedriger siedenden Bestandteile wie Wasser können aus dem Reaktionsgemisch bzw. aus dem Reaktionsgefäß entweichen bzw. die Umsetzung findet zumindest überwiegend nicht, vorzugsweise gar nicht, unter Rückflusskühlung statt. In dieser Verfahrensvariante entsteht ein Gemisch von Kondensationsprodukten, welches sich mit fortschreitender Reaktionsdauer verändert, wobei wohl die Zahl der freien Carbonsäuregruppen wie auch der freien Hydroxygruppen in dem Gemisch (wahrscheinlich durch fortschreitende Kondensationsreaktionen wie Oligomerisierungen, Polymerisierungen, Lactonbildungen etc.) laufend abnimmt. Die Abnahme der Zahl an freien Carbonsäuregruppen im Gemisch kann anhand der entsprechend laufenden Abnahme der im zeitlichen Reaktionsverlauf gemessenen vSZ-Werte verfolgt werden (vgl. auch das unten in den Beispielen angegebene Berechnungsbeispiel für die Größe "vSZ(Monoester)" für ein "offenes System").

Eigene Untersuchungen haben gezeigt, dass in dieser Verfahrensvariante ("offenes System") ein Optimum der Kombination vorteilhafter Produkteigenschaften der hergestellten oberflächenaktiven Mischungen umfassend Kondensationsprodukte - insbesondere bei den oberflächenaktiven Mischungen umfassend Kondensationsprodukte, welche gute Eigenschaften als Tenside und/oder Schaumverbesserer und/oder Schaumverstärker aufweisen - nämlich gute Wasserlöslichkeit und gute oberflächenaktive Eigenschaften, vorzugsweise gute Schaumeigenschaften (insbesondere erzielbare Schaumhöhe und/oder Schaumqualität) bei ansonsten konstant gehaltenen Parametern von der Reaktionsdauer abhängig ist. In einer bevorzugten Variante dieser Ausgestaltung ("offenes System") wird die Umsetzung für eine Dauer im Bereich von 1 bis 3 Stunden, vorzugsweise im Bereich von 1,5 bis 2,5 Stunden durchgeführt.

Es ist daher in einigen Fällen ein bevorzugtes Verfahren bevorzugt, wobei
Wasser während der Umsetzung aus dem Reaktionsgefäß entweichen kann, wobei die Umsetzung überwiegend nicht, vorzugsweise gar nicht, unter Rückflusskühlung stattfindet
und
die Reaktionsdauer im Bereich von 1 bis 3 Stunden, vorzugsweise im Bereich von 1,5 bis 2,5 Stunden liegt.

In eigenen Untersuchungen wurde in diesem Zusammenhang auch gefunden, dass die vorstehend angegebene vorhandene Säurezahl für den Monoester der eingesetzten α-Hydroxycarbonsäure mit dem eingesetzten 1,2-Alkandiol der Formel I ("vSZ(Monoester)") im Rahmen der vorliegenden Erfindung allgemein (d.h. für beide Verfahrensvarianten des "offenen Systems" sowie des "geschlossenen Systems) ein geeigneter Anhaltspunkt für die Ermittlung eines geeigneten oder des besten Zeitpunktes zur Beendigung des erfindungsgemäßen Verfahrens ist. So wiesen insbesondere die Reaktionsprodukte des erfindungsgemäßen Verfahrens, welche zu dem Zeitpunkt oder um den Zeitpunkt isoliert wurden, als der Wert der gemessenen vSZ den Wert der theoretisch ermittelten vSZ(Monoester) erreichte, eine gute oder sogar die beste Kombination von guter Wasserlöslichkeit und guten oberflächenaktiven Eigenschaften auf: war die gemessene vSZ (d.h. die durch Titration der Reaktionsprodukte ermittelte Menge in mmol an Base (OH⁻) bezogen auf eine Probe der Masse 1g der Reaktionsprodukte) deutlich höher als die theoretisch ermittelte vSZ(Monoester) (zu kurze Reaktionsdauer), so führte dies in der Regel zu schlechter wasserlöslichen Produkten mit vergleichsweise guten oberflächenaktiven Eigenschaften. War die gemessene vSZ deutlich niedriger als die theoretisch ermittelte vSZ(Monoester ) (zu lange Reaktionsdauer), so führte dies in der Regel zu besser wasserlöslichen Produkten, aber mit vergleichsweise schlechteren oberflächenaktiven Eigenschaften, insbesondere schlechteren Schaumeigenschaften.

Zur Isolierung der Verfahrensprodukte, welche eine gute oder sogar die beste Kombination von guter Wasserlöslichkeit und guten oberflächenaktiven Eigenschaften besitzen, wird das Reaktionsgemisch, vorzugsweise im Falle des "offenen Systems", daher vorzugsweise zu dem entweder durch reaktionsbegleitende Kontrolle der vSZ oder durch geeignete Vorversuche (etwa Bestimmung eines geeigneten Zeitpunkts unter vorher definierten Reaktionsbedingungen) bestimmten Zeitpunkt (Erreichen der theoretisch ermittelten vSZ(Monoester)) möglichst rasch gekühlt, vorzugsweise mit Wasser oder Eiswasser.

In der vorstehend beschriebenen Variante Verfahrens ("offenes System") wird das homogene Reaktionsgemisch vorzugsweise erzeugt, indem
(a) das 1,2-Alkandiol der Formel I (bzw. ein Gemisch solcher 1,2-Alkandiole) auf die vorgesehene Reaktionstemperatur im Bereich von 110 bis 150 °C, vorzugsweise im Bereich von 115 bis 140 °C, besonders bevorzugt im Bereich von 120 bis 135 °C, erwärmt und vorzugsweise dabei geschmolzen wird,
(b) die α-Hydroxycarbonsäure(n) in Wasser, vorzugsweise in auf eine Temperatur im Bereich von 30 bis 90 °C erwärmtem Wasser, gelöst wird
   und anschließend
(c) die in Wasser gelöste α-Hydroxycarbonsäure(n) aus Schritt (b) zu dem erwärmten 1,2-Alkandiol (bzw. dem Gemisch solcher 1,2-Alkandiole) aus Schritt (a) hinzugegeben und das resultierende Reaktionsgemisch bei der Reaktionstemperatur im Bereich von 110 bis 150 °C, vorzugsweise im Bereich von 115 bis 140 °C, besonders bevorzugt im Bereich von 120 bis 135 °C, mechanisch durchmischt wird.

In dieser vorstehend angegebenen Verfahrensvariante ("offenes System") bildet sich anfänglich eine Emulsion (flüssiges Zweiphasengemisch), welche aber in der Regel nach einer Reaktionsdauer im Bereich von 15 bis 45 Minuten in eine klare, homogene (einphasig flüssige) Phase übergeht.

In der vorstehend beschriebenen erfindungsgemäßen oder bevorzugten erfindungsgemäßen Verfahrensvariante, worin die hergestellte oberflächenaktive Mischung umfassend Kondensationsprodukte einen Anteil an nicht umgesetztem 1,2-Alkandiol von bis zu 30 Gew.-% umfasst, bezogen auf die Gesamtmasse (bzw. das Gesamtgewicht) des als Ausgangsverbindung eingesetzten 1,2-Alkandiols der Formel I, ist das nicht umgesetzte 1,2-Alkandiol vorzugsweise unverzweigtes n-1,2-Decandiol. In eigenen Untersuchungen wurde gefunden, dass eine nach dieser Verfahrensvariante hergestellte oberflächenaktive Mischung umfassend Kondensationsprodukte besonders gute Schaumeigenschaften (insbesondere besonders gute erzielbare Schaumhöhe und/oder Schaumqualität) aufwies.

Weiterhin bevorzugt ist ein erfindungsgemäßes oder bevorzugtes erfindungsgemäßes Verfahren,
wobei
Citronensäure
mit mindestens einem 1,2-Alkandiol der Formel I:

   R¹-CH(OH)-CH₂-OH (I),
worin
R¹ eine lineare oder verzweigte, vorzugsweise eine lineare, Kohlenwasserstoffgruppe mit insgesamt 6 bis 18 Kohlenstoffatomen, vorzugsweise eine lineare Kohlenwasserstoffgruppe mit insgesamt 6 bis 16 Kohlenstoffatomen und besonders bevorzugt eine lineare Kohlenwasserstoffgruppe mit insgesamt 8 bis 14 Kohlenstoffatomen, bedeutet,
   - in einem molaren Verhältnis des 1,2-Alkandiols zu der Citronensäure im Bereich von 1 : 0,5 bis 1: 2,5, vorzugsweise im Bereich von 1 : 1 bis 1: 2, besonders bevorzugt im Bereich von 1 : 1,5 bis 1: 2,
   - bei einer Temperatur im Bereich von 110 bis 150 °C, vorzugsweise im Bereich von 115 bis 140 °C, besonders bevorzugt im Bereich von 120 bis 135 °C, und
   - in Anwesenheit von Wasser
umgesetzt wird,
wobei die Umsetzung vorzugsweise zumindest zeitweilig, besonders bevorzugt andauernd über den gesamten Reaktionsverlauf, in einem homogenen Reaktionsgemisch durchgeführt wird
   und
wobei Wasser während der Umsetzung zumindest zeitweilig, vorzugsweise andauernd über den gesamten Reaktionsverlauf, am Entweichen aus dem Reaktionsgefäß gehindert wird, wobei vorzugsweise die Umsetzung zumindest zeitweilig unter Rückflusskühlung durchgeführt wird
   und
wobei vorzugsweise die Reaktionsdauer im Bereich von 3 bis 7 Stunden, besonders bevorzugt im Bereich von 4 bis 6, Stunden liegt.

Die Erfindung betrifft ebenfalls eine oberflächenaktive Mischung umfassend Kondensationsprodukte, herstellbar oder hergestellt nach einem vorstehend angegebenen erfindungsgemäßen oder bevorzugten erfindungsgemäßen Verfahren.

Alle vorstehend für das erfindungsgemäße Verfahren zur Herstellung einer oberflächenaktiven Mischung umfassend Kondensationsprodukte angegebenen erfindungsgemäßen Ausgestaltungen und Kombinationen, einschließlich der jeweils als bevorzugt angegebenen Ausgestaltungen und Kombinationen, gelten ohne Einschränkung und gegebenenfalls sinngemäß auch für die vorstehend angegebene erfindungsgemäße oberflächenaktive Mischung.

In einer bevorzugten Ausgestaltung der vorstehend angegebenen erfindungsgemäßen oberflächenaktiven Mischung umfassend Kondensationsprodukte ist diese herstellbar oder hergestellt nach einem vorstehend angegebenen erfindungsgemäßen oder bevorzugten erfindungsgemäßen Verfahren, worin R¹ in dem 1,2-Alkandiol der Formel I eine lineare Kohlenwasserstoffgruppe mit insgesamt 6 bis 12 Kohlenstoffatomen, vorzugsweise mit insgesamt 8 bis 10 Kohlenstoffatomen, bedeutet und besonders bevorzugt n-1,2-Decandiol ist. Es wurde in eigenen Versuchen festgestellt, dass diese spezifische Mischung besonders gute Eigenschaften als Tensid und/oder Schaumverbesserer und/oder Schaumverstärker aufweist. Besonders bevorzugt sind solche hier vorstehend genannten bevorzugten oberflächenaktiven Mischungen umfassend Kondensationsprodukte, worin als α-Hydroxycarbonsäure Citronensäure enthalten ist und/oder eingesetzt wurde.

In einer weiteren bevorzugten Ausgestaltung der vorstehend angegebenen erfindungsgemäßen oberflächenaktiven Mischung umfassend Kondensationsprodukte ist diese herstellbar oder hergestellt nach einem vorstehend angegebenen erfindungsgemäßen oder bevorzugten erfindungsgemäßen Verfahren, worin R¹ in dem 1,2-Alkandiol der Formel I eine lineare Kohlenwasserstoffgruppe mit insgesamt 14 bis 18 Kohlenstoffatomen, vorzugsweise mit insgesamt 14 bis 16 Kohlenstoffatomen und besonders bevorzugt mit insgesamt 14, Kohlenstoffatomen bedeutet. Es wurde in eigenen Versuchen festgestellt, dass diese spezifische Mischung besonders gute Eigenschaften als Emulgator aufweist. Besonders bevorzugt sind solche hier vorstehend genannten bevorzugten oberflächenaktiven Mischungen umfassend Kondensationsprodukte, worin als α-Hydroxycarbonsäure Citronensäure oder Äpfelsäure enthalten ist bzw. eingesetzt wurde.

Besonders bevorzugt ist eine Ausgestaltung der vorstehend angegebenen erfindungsgemäßen oberflächenaktiven Mischung umfassend Kondensationsprodukte, umfassend
- Kondensationsprodukte der Umsetzung der α-Hydroxycarbonsäure, vorzugsweise Citronensäure, mit dem 1 ,2-Alkandiol der Formel I, vorzugsweise n-1 ,2-Decandiol,
- nicht umgesetztes 1 ,2-Alkandiol der Formel I, vorzugsweise n-1 ,2-Decandiol, und
- Wasser,
wobei vorzugsweise der Anteil an nicht umgesetztem 1 ,2-Alkandiol der Formel I bis zu 30 Gew.-%, vorzugsweise im Bereich von 10 bis 25 Gew.-%, beträgt, bezogen auf die Gesamtmasse (bzw. das Gesamtgewicht) des als Ausgangsverbindung eingesetzten1,2-Al-kandiols der Formel I.

Es wurde in eigenen Untersuchungen gefunden, dass eine vorstehend angegebene oberflächenaktive Mischung umfassend Kondensationsprodukte, insbesondere in ihren als bevorzugt angegebenen Ausgestaltungen, besonders gute Schaumeigenschaften (insbesondere erzielbare Schaumhöhe und/oder Schaumqualität) aufweist.

Die vorliegende Erfindung betrifft auch die Verwendung einer vorstehend angegebenen erfindungsgemäßen oder bevorzugten erfindungsgemäßen oberflächenaktiven Mischung umfassend Kondensationsprodukte als Bestandteil oder einzigen Bestandteil eines Wasch- oder Reinigungsmittels und/oder eines Kosmetikproduktes.

Als Wasch- oder Reinigungsmittel kommen insbesondere Spül- oder Waschmittel für Haushalt, Körperhygiene oder persönliche Körperpflege ("personal care") in Frage, etwa Geschirrspülmittel, Wasch- bzw. Reinigungsmittel für die Wäschepflege, Seifen, Duschgels und/oder Waschlotionen. Als Kosmetikprodukte kommen insbesondere Haar- und Hautpflegeprodukte wie Hautcremes, Hautlotionen oder Haarshampoos in Frage.

Alle vorstehend für das erfindungsgemäße Verfahren zur Herstellung einer oberflächenaktiven Mischung umfassend Kondensationsprodukte und/oder für die erfindungsgemäße oberflächenaktive Mischung umfassend Kondensationsprodukte angegebenen erfindungsgemäßen Ausgestaltungen und Kombinationen, einschließlich der jeweils als bevorzugt angegebenen Ausgestaltungen und Kombinationen, gelten ohne Einschränkung und gegebenenfalls sinngemäß auch für die vorstehend angegebene erfindungsgemäße Verwendung einer erfindungsgemäßen oberflächenaktiven Mischung umfassend Kondensationsprodukte.

Bevorzugt ist eine vorstehend angegebene erfindungsgemäße Verwendung einer erfindungsgemäßen oberflächenaktiven Mischung umfassend Kondensationsprodukte, welche herstellbar oder hergestellt ist nach einem vorstehend angegebenen erfindungsgemäßen oder bevorzugten erfindungsgemäßen Verfahren,
worin
R¹ in dem 1,2-Alkandiol der Formel I eine lineare Kohlenwasserstoffgruppe mit insgesamt 6 bis 18 Kohlenstoffatomen, vorzugsweise mit insgesamt 6 bis 16 Kohlenstoffatomen, besonders bevorzugt mit insgesamt 8 bis 14 Kohlenstoffatomen, bedeutet,
und worin vorzugsweise
   R¹ in dem 1,2-Alkandiol der Formel I eine lineare Kohlenwasserstoffgruppe mit insgesamt 6 bis 12 Kohlenstoffatomen, vorzugsweise mit insgesamt 8 bis 10 Kohlenstoffatomen, bedeutet und besonders bevorzugt der oder mindestens ein 1,2-Alkandiol der Formel I n-1 ,2-Decandiol ist,
als Tensid und/oder Schaumverbesserer und/oder Schaumverstärker.

Als Tensid und/oder Schaumverbesserer und/oder Schaumverstärker bevorzugt geeignete erfindungsgemäße oberflächenaktive Mischungen umfassend Kondensationsprodukte weisen insbesondere (1) eine gute Wasserlöslichkeit und/oder (2) eine gute erzielbare Schaumhöhe und/oder (3) eine gute erzielbare Schaumqualität auf. Vorzugsweise weisen solche als Tensid und/oder Schaumverbesserer und/oder Schaumverstärker bevorzugt geeignete erfindungsgemäße oberflächenaktive Mischungen umfassend Kondensationsprodukte eine, zwei oder besonders bevorzugt alle drei der vorgenannten Eigenschaften auf.

Bevorzugt ist ebenfalls eine vorstehend angegebene erfindungsgemäße Verwendung einer erfindungsgemäßen oberflächenaktiven Mischung umfassend Kondensationsprodukte, welche herstellbar oder hergestellt ist nach einem vorstehend angegebenen erfindungsgemäßen oder bevorzugten erfindungsgemäßen Verfahren,
worin
R¹ in dem 1 ,2-Alkandiol der Formel I eine lineare Kohlenwasserstoffgruppe mit insgesamt 6 bis 18 Kohlenstoffatomen, vorzugsweise mit insgesamt 6 bis 16 Kohlenstoffatomen, besonders bevorzugt mit insgesamt 8 bis 14 Kohlenstoffatomen, bedeutet,
und worin vorzugsweise
   R¹ in dem 1 ,2-Alkandiol der Formel I eine lineare Kohlenwasserstoffgruppe mit insgesamt 14 bis 18 Kohlenstoffatomen, vorzugsweise mit insgesamt 14 bis 16 Kohlenstoffatomen, besonders bevorzugt mit insgesamt 14, Kohlenstoffatomen bedeutet,
als Emulgator.

Als Emulgatoren bevorzugt geeignete erfindungsgemäße oberflächenaktive Mischungen umfassend Kondensationsprodukte eignen sich insbesondere zur Herstellung von Emulsionen, welche sich durch eine hohe Stabilität (d.h. zeitlich lang anhaltende Stabilität über vorzugsweise mehrere Wochen, besonders bevorzugt über nicht weniger als acht Wochen) auszeichnen.

Die vorliegende Erfindung betrifft weiterhin ein Wasch- oder Reinigungsmittel und/oder ein Kosmetikprodukt, enthaltend eine vorstehend beschriebene erfindungsgemäße oder bevorzugte erfindungsgemäße oberflächenaktive Mischung umfassend Kondensationsprodukte. Als Wasch- oder Reinigungsmittel bzw. als Kosmetikprodukte kommen insbesondere die vorstehend angegebenen Waschmittel, Reinigungsmittel bzw. Kosmetikprodukte in Frage.

Alle vorstehend für das erfindungsgemäße Verfahren zur Herstellung einer oberflächenaktiven Mischung umfassend Kondensationsprodukte und/oder für die erfindungsgemäße oberflächenaktive Mischung umfassend Kondensationsprodukte und/oder für die erfindungsgemäße Verwendung angegebenen erfindungsgemäßen Ausgestaltungen und Kombinationen, einschließlich der jeweils als bevorzugt angegebenen Ausgestaltungen und Kombinationen, gelten ohne Einschränkung und gegebenenfalls sinngemäß auch für das vorstehend angegebene erfindungsgemäße Wasch- oder Reinigungsmittel und/oder Kosmetikprodukt.

### Figuren:

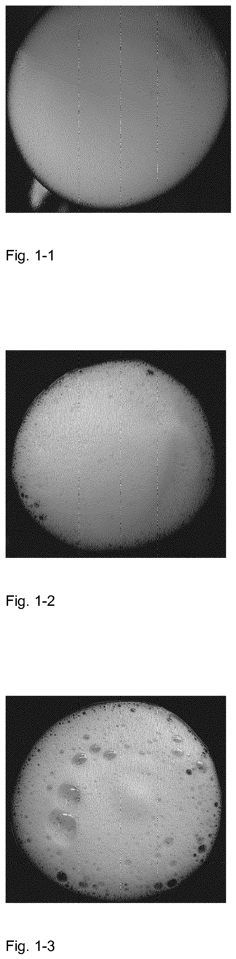

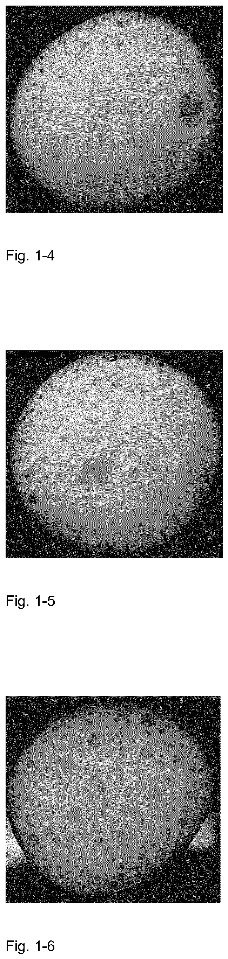

Die Figuren 1-1 bis 1-6 zeigen Musteransichten für die Bewertung bzw. Beurteilung von Schaumqualitäten gemäß Beispiel 5 (Schaumtest):
- Fig. 1-1:: Schaumqualität sehr gut (1), feinporig, keine großen Luftblasen erkennbar
- Fig. 1-2:: Schaumqualität gut (2), im Wesentlichen feinporig, wenige größere Luftblasen erkennbar;
- Fig. 1-3:: Schaumqualität befriedigend (3), teils feinporig, teils größere Luftblasen erkennbar, wenige große Luftblasen;
- Fig. 1-4:: Schaumqualität ausreichend (4), mehr größere Luftblasen als feinporiger Schaum, wenige große Luftblasen;
- Fig. 1-5:: Schaumqualität nicht ausreichend (5), größere Luftblasen deutlich in der Mehrzahl, wenige große Luftblasen;
- Fig. 1-6:: Schaumqualität mangelhaft (6), fast ausnahmslos größere und große Luftblasen.

### Beispiele:

Die nachfolgenden Beispiele sollen die Erfindung weiter beschreiben und erklären, ohne ihren Umfang zu beschränken.

Soweit nicht anders angegeben, wurden alle nachfolgend angegebenen Versuche unter Normalbedingungen durchgeführt (Raumtemperatur: 20 °C, Druck: 1013,25 hPa).

Zum Rühren (mechanische Durchmischung) der Reaktionsmischungen nach der Methode des nachfolgend angegebenen Beispiels 1 wurde ein Rührmotor mit Rühranker aus Edelstahl verwendet. Zum Rühren (mechanische Durchmischung) der Reaktionsmischungen nach der Methode des nachfolgend angegebenen Beispiels 2 wurde ein herkömmlicher Magnetrührer verwendet.

Zur Messung des pH-Wertes (vgl. Beispiele 4, 5) wurde ein pH-Meter "WTW 330" der Firma Wissenschaftlich-Technische Werkstätten GmbH verwendet, mit Elektrode "SenTix61" und Temperaturfühler.

Zur Durchführung der Schaumtests (Beispiel 5) wurde ein Rührmotor vom Typ "Eurostar 20 digital" der Firma IKA-Werke GmbH & Co, KG, Deutschland verwendet, der mit einer Dispersionsscheibe ("Z-Scheibe", Durchmesser 6 cm) ausgestattet war ("Rührwerk").

In den Beispielen werden die folgenden Abkürzungen mit den nachfolgend angegebenen Bedeutungen verwendet:
- CS:: Citronensäure
- ÄS:: D,L-Äpfelsäure
- WS:: L-Weinsäure
- vSZ gem.:: gemessene "vorhandene Säurezahl" nach Reaktionsdauer (vgl. Beispiel 3);
- vSZ theor.:: theoretische "vorhandene Säurezahl" nach Reaktionsdauer (vgl. Beispiel 3) unter der Annahme, dass bei der Reaktion quantitativ der Monoester der Citronensäure mit dem jeweils eingesetzten 1,2-Alkandiol der Formel I gebildet wurde (entspricht der Größe "vSZ(Monoester)");
- S_{H}:: Schaumhöhe (vgl. Beispiel 5)
- S_{Q}:: Schaumqualität (vgl. Beispiel 5)
- L (H₂O):: Löslichkeit in Wasser (vgl. Beispiel 4)
- n:: Stoffmenge (in mmol)
- T:: Temperatur
- t Rkt.:: Reaktionsdauer
- tₘₐₓ Stabilität: Emulsion: längste beobachtete Zeitdauer, während der eine Emulsion stabil war (planmäßiges Versuchsende nach 10 Wochen)
- k.A.:: keine Angabe
- C10:: n-1,2-Decandiol
- C12:: n-1,2-Dodecandiol
- C16:: n-1,2-Hexadecandiol

Die Ausgangsstoffe n-1,2-Decandiol und n-1,2-Dodecandiol sind kommerziell erhältlich und wurden als Fertigprodukte gekauft (TCI Deutschland GmbH).

Der Ausgangsstoff n-1 ,2-Hexadecandiol ist ebenfalls kommerziell erhältlich, wurde aber für die Zwecke der vorliegenden Erfindung auf an sich bekannte Weise aus kommerziell erhältlichem 1,2-Epoxyhexadecan durch säurekatalysierte Addition von Wasser selbst hergestellt und nach Umkristallisation aus Essigsäureethylester direkt für die nachfolgend angegebenen Synthesen eingesetzt.

### Beispiel 1: Herstellung von erfindungsgemäßen oberflächenaktiven Mischungen umfassend Kondensationsprodukte (Verfahrensvariante "offenes System") (nicht erfindungsgemäßes Verfahren)

Es wurden erfindungsgemäße oberflächenaktive Mischungen umfassend Kondensationsprodukte nach der folgenden, allgemeinen Synthesevorschrift hergestellt:
Die jeweils gewünschte (molare) Menge an 1,2-Alkandiol der Formel I (bzw. einem Gemisch solcher 1 ,2-Alkandiole) wird in einem offenen Reaktionsgefäß (z.B. Becherglas) mittels einer externen Heizvorrichtung (z.B. Ölbad) auf die jeweils gewünschte Reaktionstemperatur im Bereich von 110 bis 150 °C erwärmt (und dabei in der Regel geschmolzen). Die jeweils gewünschte Menge der α-Hydroxycarbonsäure mit 3 bis 6 Kohlenstoffatomen (bzw. eines Gemisches solcher α-Hydroxycarbonsäuren) wird in der Hitze (z.B. bei einer Temperatur im Bereich von 30 bis 60 °C) mit etwas Wasser gelöst. Die erhitzte Lösung der α-Hydroxycarbonsäure(n) in Wasser wird unter Rühren mit einem geeigneten Rührer (z.B. Ankerrührer) langsam zur Vorlage des heißen 1,2-Alkandiols der Formel I hinzugegeben und die entstandene Reaktionsmischung wird für die gewünschte Reaktionsdauer bei der gewünschten Reaktionstemperatur weiter gerührt. Zum Beenden der Reaktion werden Rührer und Ölbad entfernt und das erhaltene Reaktionsprodukt (erfindungsgemäße oberflächenaktive Mischungen umfassend Kondensationsprodukte) wird noch heiß in ein Probengefäß überführt.

Zur Untersuchung des Reaktionsfortschrittes bzw. der (unten näher beschriebenen) Produkteigenschaften (vgl. Beispiele 3 bis 5) wurden jeweils Proben aus dem Reaktionsgefäß entnommen.

Gemäß der vorstehend angegebenen allgemeinen Synthesevorschrift hergestellte erfindungsgemäße oberflächenaktive Mischungen umfassend Kondensationsprodukte, bzw. die zu deren Herstellung verwendeten Parameter, sind in der nachfolgend angegebenen Tabelle 2a bzw. 2b aufgeführt.

### Beispiel 2: Herstellung von erfindungsgemäßen oberflächenaktiven Mischungen umfassend Kondensationsprodukte (Verfahrensvariante "geschlossenes System")

Es wurden erfindungsgemäße oberflächenaktive Mischungen umfassend Kondensationsprodukte nach der folgenden, allgemeinen Synthesevorschrift hergestellt:
In einem Rundkolben mit Rückflusskühler und Druckausgleich wird die jeweils gewünschte (molare) Menge an α-Hydroxycarbonsäure mit 3 bis 6 Kohlenstoffatomen (bzw. eines Gemisches solcher α-Hydroxycarbonsäuren) unter Rühren mit einem geeigneten Rührer (z.B. Magnetrührer) in wenig Wasser gelöst und mittels einer externen Heizvorrichtung (z.B. Ölbad) erwärmt. Zu dieser Vorlage wird die jeweils gewünschte (molare) Menge an 1,2-AIkandiol der Formel I (bzw. einem Gemisch solcher 1,2-Alkandiole) hinzugegeben und die entstandene Reaktionsmischung wird unter Rückflusskühlung für die gewünschte Reaktionsdauer bei der gewünschten Reaktionstemperatur im Bereich von 110 bis 150 °C weiter gerührt. Zum Beenden der Reaktion werden Rührer und Ölbad entfernt und das erhaltene Reaktionsprodukt (erfindungsgemäße oberflächenaktive Mischungen umfassend Kondensationsprodukte) wird noch heiß in ein Probengefäß überführt.

Zur Untersuchung der (unten näher beschriebenen) Produkteigenschaften (vgl. Beispiele 3 bis 5) werden nach Beenden der Reaktion jeweils Proben aus dem Reaktionsgefäß entnommen.

Gemäß der vorstehend angegebenen allgemeinen Synthesevorschrift hergestellte erfindungsgemäße oberflächenaktive Mischungen umfassend Kondensationsprodukte, bzw. die zu deren Herstellung verwendeten Parameter, sind in der nachfolgend angegebenen Tabelle 2a bzw. 2b aufgeführt.

### Beispiel 3: Bestimmung der "vorhandenen Säurezahl" ("vSZ")

Zur Abschätzung des Reaktionsfortschrittes des erfindungsgemäßen Verfahrens bzw. zur Bestimmung des geeigneten Zeitpunktes zur Beendigung der Reaktion, wurde die "vorhandene Säurezahl" von erfindungsgemäßen oberflächenaktiven Mischungen umfassend Kondensationsprodukte (nachfolgend angegeben als "Testsubstanz(en)") gemäß nachfolgend beschriebener Testmethode bestimmt:
Die "vorhandene Säurezahl" (in Einheiten von mmol OH⁻/g Testsubstanz) gibt an, wie viele Säureäquivalente sich in einem Gramm Testsubstanz befinden (s. auch vorstehende Erklärungen hierzu).

Zur Bestimmung der "vorhandenen Säurezahl" ("vSZ") der Testsubstanzen wurden diese als Feststoffe direkt aus dem Reaktionsgefäß entnommen, ohne weitere Behandlung abgewogen und - je nach Löslichkeit - in 20 - 40 ml Wasser, einem Wasser/Methanol-Gemisch bzw. einem Wasser/Ethanol-Gemisch gelöst, suspendiert oder emulgiert und dann mit wässriger NaOH (c = 0,1 mol/l) bis zum Farbumschlag von Phenolphthalein (farblos nach rosa) titriert. Der Verbrauch an Natronlauge entspricht den Säureäquivalenten und damit der vorhandenen Säurezahl. Sofern beispielsweise Citronensäure (wasserfrei) und deren Mono- (M = 348,4 g/mol), Di- (M = 504,7 g/mol) bzw. Triester mit n-1,2-Decandiol (M = 660,9 g/mol) nach dem vorstehend angegeben Verfahren untersucht werden, betragen die theoretisch erwarteten vorhandenen Säurezahlen:

| | |
|---|---|
| Für den Triester: | vSZ_{theor.} = 0 (keine titrierbaren Carbonsäuregruppen) |
| Für den Diester: | vSZ_{theor.} = 1,98 mmol/g |
| Für den Monoester: | vSZ_{theor.} = 5,74 mmol/g |

Für die folgenden Testsubstanzen gemäß unten angegebener Tabelle 2a bzw. 2b wurden die ebenfalls in Tabelle 2a bzw. 2b angegebenen "vorhandenen Säurezahlen" nach den ebenfalls in Tabelle 2a bzw. 2b angegebenen Reaktionszeiten (Reaktionsdauer) gemessen. Als Testsubstanzen wurden jeweils die oberflächenaktiven Mischungen umfassend Kondensationsprodukte verwendet, wie sie nach Beendigung der Reaktion (Ende der Reaktionsdauer t Rkt.) nach Durchführung des erfindungsgemäßen Verfahrens vorlagen, d.h. ohne weitere Reinigung oder Aufarbeitung. Nachfolgend werden Berechnungsbeispiele für die Größen "vSZ theor." bzw. "vSZ(Monoester)" für das "offene" und für das "geschlossene" System entsprechend dem erfindungsgemäßen Verfahren angegeben.

Berechnungsbeispiel 1 - für "vSZ theor." bzw, "vSZ(Monoester)" zu Beispiel 1 (Tabelle 2a), Variante offenes System:
Edukte: 180 mmol Citronensäure (m = 34,76 g; 540 mmol Säureäquivalente) + 120 mmol 1,2-Decandiol (m = 20,91 g; 0 mol Säureäquivalente);
Produkte (theoretisch): 120 mmol Monoester von Citronensäure mit 1,2-Decandiol (41,81 g; 240 mmol Säureäquivalente) + 60 mmol Citronensäure (11,52 g; 180 mmol Säureäquivalente) (+ 120 mmol H₂O).
vSZ_{theor.} = 420 mmol Säureäquivalente / 53,33 g = 7,9 mmol/g.

Berechnungsbeispiel 2 - für "vSZ theor." bzw, "vSZ(Monoester)" zu Beispiel 7 (Tabelle 2a), Variante geschlossenes System:
Edukte: 120 mmol Citronensäure-Monohydrat (m = 25,2 g; 360 mmol Säureäquivalente) + 60 mmol 1,2-Decandiol (m = 10,5 g; 0 mmol Säureäquivalente) + 5,0 g Wasser; Eingesetzte Gesamtmasse: 40,7 g.
Produkte (theoretisch): 60 mmol Monoester von Citronensäure mit 1,2-Decandiol (120 mmol Säureäquivalente) + 60 mmol Citronensäure (180 mmol Säureäquivalente).
vSZ_{theor.} = 300 mmol Säureäquivalente / 40,7 g = 7,4 mmol/g.

### Beispiel 4: Löslichkeitstest

Die Wasserlöslichkeit von erfindungsgemäßen oberflächenaktiven Mischungen umfassend Kondensationsprodukte (nachfolgend angegeben als "Testsubstanz(en)") wurde gemäß nachfolgend beschriebener Testmethode bestimmt.

Es wurden jeweils 2,4 g einer Testsubstanz in 10 ml vollentionisiertem Wasser gelöst, suspendiert oder emulgiert (je nach Löslichkeit) und mit einer geeigneten Menge einer wässrigen NaOH-Lösung (c = 0,5 mol/l) auf einen pH-Wert zwischen 5,5 und 6,0 eingestellt (pH-Meter) und dann mit vollentionisiertem Wasser auf ein Gesamtgewicht der Probe von 20,0 g aufgefüllt. Nicht klar gelöste Proben wurden 15 bis 20 min. lang im Ultraschallbad behandelt oder auf 40 bis 50 °C erwärmt (max. 1 h auf 40 °C, max. 30 min. auf 50 °C) und 3 bis 4 h bei Raumtemperatur stehen gelassen. Anschließend wurde die entstandene Mischung visuell bewertet auf das Vorliegen einer klaren Lösung bzw. einer Trübung.

Die Ergebnisse des Löslichkeitstests "+" als gut/klar löslich, "(+)" als recht gut / leicht getrübt löslich und "-" als schlecht / nur mit deutlicher Trübung löslich) für verschiedene Testsubstanzen sind in der nachfolgend angegebenen Tabelle 2a bzw. 2b aufgeführt.

### Beispiel 5: Schaumtest

Die Fähigkeit von erfindungsgemäßen oberflächenaktiven Mischungen umfassend Kondensationsprodukte (nachfolgend angegeben als "Testsubstanz(en)"), als Tensid bzw. Schaumbildner und/oder Schaumverstärker zu wirken, wurde gemäß nachfolgend beschriebener Testmethode bestimmt:
In einem 800 ml-Becherglas (Schott Duran, H: 13,5 cm, R: 9,4 cm) mit Rührwerk wurden jeweils 2,5 g einer Testsubstanz eingewogen und mit 50 ml Leitungswasser versetzt. Die entstandene wässrige Mischung bzw. Lösung wurde durch Zugabe einer geeigneten Menge von wässriger NaOH-Lösung (c = 1,0 mol/l) auf einen pH-Wert von 5,5 eingestellt, mit warmem Wasser (ca. 30 °C) auf 100 ml Gesamtvolumen aufgefüllt und dann auf 30° C temperiert (Wasserbad). Anschließend wurde die entstandene, temperierte Mischung zur Schaumerzeugung 10 s lang (Stoppuhr) bei einer Umdrehungszahl von 2000 U/min gerührt.

Unmittelbar nach Abstellen des Rührmotors und ohne den Rührer (Dispersionsscheibe, s.o.) aus dem Becherglas zu entfernen, wurde anschließend die Höhe des entstandenen Schaumes vom Boden des Becherglases aus mit einem Lineal gemessen und als "Schaumhöhe" ("S_{H}") in cm angegeben.

Danach wurde mit einem Löffel eine Probe des entstandenen Schaumes entnommen, auf einen dunklen Untergrund aufgebracht und die Schaumqualität wurde visuell (Doppelbestimmung) mit Werten auf einer Skala von 1 bis 6 ("1": sehr gut, "6": mangelhafte Schaumqualität) bewertet. Beispiele für die Bewertung von Schaumqualitäten sind in den beigefügten Zeichnungen (vgl. Fig. 1-1 bis 1-6) abgebildet. Als Standard (S_{Q} = 2) diente dabei das kommerziell erhältliche Tensid Texapon^{®} NSO (Natriumlaurylethersulfat).

Die Ergebnisse des Schaumtests (Schaumhöhen "S_{H}", in cm, und Schaumqualitäten "S_{Q}", in Werten von 1 bis 6, Mittelwert aus jeweils drei Messungen) für verschiedene Testsubstanzen sind in der nachfolgend angegebenen Tabelle 2a aufgeführt. Besonders gute Eigenschaften als Tenside bzw. Schaumbildner und/oder Schaumverstärker zeigten die Beispiel-Verbindungen, welche eine Schaumhöhe von 8,5 cm oder darüber und/oder (vorzugsweise "und") eine Schaumqualität von mindestens 3 oder besser aufwiesen.

### Beispiel 6: Emulsionstest

Die Fähigkeit von erfindungsgemäßen oberflächenaktiven Mischungen umfassend Kondensationsprodukte (nachfolgend angegeben als "Testsubstanz(en)"), als Emulgator zu wirken (d.h. stabile Emulsionen zu bilden), wurde gemäß nachfolgend beschriebener Testmethode bestimmt:
Es wurden pro Testsubstanz jeweils 50,0 g einer Emulsion mit folgenden Inhaltsstoffen hergestellt (vgl. Tabelle 1):

**Tabelle 1: Bestandteile einer Test-Emulsion**

| **Nr.** | **Bestandteil** | **Gew.-%** | **Anmerkung** |
|---|---|---|---|
| 1 | Testsubstanz | 3 | Vgl. Tabelle 3b |
| 2 | Mischung aus Hexadecanol und Octadecanol | 5 | Lanette^{®} O (BASF SE); CAS RN 67762-27-0 |
| 3 | Kokosfett-Kokosalkoholester / Triglycerid-Öl | 17 | Cetiol^{®} LC (BASF SE); CAS RN 95912-86-0 |
| 4 | Wasser (+NaOH) | 75 | ./. |
| ./. | SUMME | 100 | ./. |

Hierzu wurden die Bestandteile 1, 2 und 3 der vorstehend angegebenen Test-Emulsion (s. Tabelle 1) miteinander vermischt und die entstandene Mischung wurde auf 70 °C erwärmt. Anschließend wurde die so erhaltene Mischung mit der entsprechenden Menge auf 70 °C erwärmten Wassers versetzt, dem vorher eine ausreichende Menge einer wässrigen NaOH-Lösung (c = 1,0 mol/l) zugesetzt worden war (Bestandteil 4), so dass die fertige Test-Emulsion einen pH-Wert im Bereich von 5,5 bis 6 aufwies. Zur Herstellung der fertigen Test-Emulsion wurde die Mischung aller vier Bestandteile dann für ca. 20 s mit einem Hochleistungs-Dispergiergerät vom Typ "Ultra-Turrax^{®} der Firma IKA-Werke GmbH & Co KG bei einer Drehzahl von 20000 U/min homogenisiert.

Die auf diese Weise hergestellten Test-Emulsionen wurden noch warm (bei einer Temperatur von ≥ 50°C) in Probenbehälter abgefüllt und dann bei einer Temperatur von 40 °C (Trockenschrank) für jeweils bis zu zehn Wochen gelagert. Während der Lagerzeit wurden die Proben jeweils wöchentlich visuell auf ihre Stabilität untersucht: War bei visueller Prüfung die Abscheidung von Wasser (als Tröpfchen) zu beobachten, wurde die Emulgierfähigkeit der betrachteten Testsubstanz über den geprüften Zeitraum als negativ ("-") bewertet. War bei der visuellen Prüfung keine Abscheidung von Wasser in der Test-Emulsion zu erkennen, wurde die Emulgierfähigkeit der betrachteten Testsubstanz über den geprüften Zeitraum als positiv ("+") bewertet.

Die Ergebnisse des Emulsionstests für verschiedene Testsubstanzen sind in der nachfolgend angegebenen Tabelle 2b als Bewertungen der Stabilität der Emulsionen über einen jeweils in Tabelle 2b angegebenen Zeitraum aufgeführt.

**Tabelle 2a: Übersicht über erfindungsgemäß hergestellte oberflächenaktive Mischungen umfassend Kondensationsprodukte**

| Nr. | Verfahren gemäß Beispiel | Säure | n (Säure) [mmol] | 1,2-Alkandiol | n (1.2-Alkandiol) [mmol] | Wasser [ml] | T (Ölbad) [°C] | t Rkt. [min.] | vSZ gem. [mmol/g] | vSZ theor. [mmol/g] | S_{H} [cm] | S_{Q} | L(H₂O) |
|---|---|---|---|---|---|---|---|---|---|---|---|---|---|
| 1 | 1 | CS | 180 | C10 | 120 | 30 | 130 | 60 | 8,9 | 7,9 | 9,5 | 2 | - |
| 2 | 1 | CS | 180 | C10 | 120 | 30 | 130 | 120 | 7,6 | 7,9 | 9,1 | 2-3 | + |
| 3 | 1 | CS | 240 | C10 | 120 | 30 | 130 | 90 | 8,3 | 9,3 | 9,9 | 2 | + |
| 4 | 1 | CS | 240 | C10 | 120 | 30 | 130 | 120 | 7,4 | 9,3 | 9,3 | 2 | + |
| 5 | 1 | CS | 240 | C10 | 120 | 30 | 130 | 80 | 9,1 | 9,3 | 9,9 | 2 | (+) |
| 6 | 1 | CS | 240 | C10 | 120 | 40 | 120 | 90 | 9,0 | 9,3 | k.A. | k.A. | - |
| 7 | 2 | CS | 120 | C10 | 60 | 5 | 130 | 300 | 7,5 | 7,4 | k.A. | k.A. | + |
| 8 | 1 | ÄS | 400 | C10 | 267 | 50 | 115 | 300 | 4,4 | 5,6 | k.A. | k.A. | (+) |
| 9 | 2 | ÄS | 120 | C10 | 60 | 5 | 130 | 300 | 5,8 | k.A. | k.A. | k.A. | - |
| 10 | 2 | WS | 120 | C10 | 60 | 5 | 130 | 300 | 5,4 | k.A. | k.A. | k.A. | - |
| 11 | 2 | ÄS/WS | 60/60 | C10 | 60 | 5 | 130 | 300 | 6,6 | k.A. | k.A. | k.A. | - |
| 12 | 1 | CS | 180 | C12 | 120 | 30 | 130 | 60 | 8,7 | 7,4 | 9,0 | 3 | - |

| Nr. | Verfahren gemäß Beispiel | Säure | n (Säure) [mmol] | 1,2-Alkandiol | n (1,2-AIkandiol) [mmol] | Wasser [ml] | T (Ölbad) [°C] | t Rkt. [min.] | vSZ gem. [mmol/g] | vSZ theor. [mmol/g] | S_{H} [cm] | So | L (H₂O) |
|---|---|---|---|---|---|---|---|---|---|---|---|---|---|
| 13 | 1 | CS | 180 | C12 | 120 | 30 | 130 | 120 | 7,1 | 7,4 | 8,6 | 2 | + |
| 14 | 1 | CS | 240 | C12 | 120 | 30 | 130 | 90 | 8,7 | 8,8 | 8,6 | 2 | + |
| 15 | 1 | CS | 180 | C12 | 120 | 30 | 115 | 180 | 8,3 | 7,4 | 8,7 | 3 | - |
| 16 | 2 | CS | 60 | C12 | 120 | 5 | 140 | 120 | 7,6 | 7,1 | k.A. | k.A. | - |

**Tabelle 2b: Übersicht über erfindungsgemäß hergestellte oberflächenaktive Mischungen umfassend Kondensationsprodukte**

| Nr. | Verfahren gemäß Beispiel. | Säure | n (Säure) [mmol] | 1,2-Alkandiol | n (1,2-Alkandiol) [mmol] | Wasser [ml] | T (Ölbad) [°C] | t Rkt. [min.] | vSZ gem. [mmol/g] | tₘₐₓ Stabilität Emulsion [Wochen] |
|---|---|---|---|---|---|---|---|---|---|---|
| 17 | 1 | CS | 120 | C16 | 80 | 18 | 130 | 60 | 6,5 | 10 |
| 18 | 1 | CS | 120 | C16 | 80 | 18 | 130 | 120 | 5,9 | 10 |
| 19 | 2 | CS | 80 | C16 | 40 | 3,3 | 130 | 150 | 6,8 | 10 |
| 20 | 2 | CS | 80 | C16 | 40 | 3,3 | 130 | 300 | 5,5 | *10 |
| 21 | 2 | CS | 90 | C16 | 60 | 3,7 | 130 | 300 | 5,6 | 10 |
| 22 | 2 | CS | 80 | C16 | 80 | 3,3 | 130 | 300 | 4,5 | 10 |
| 23 | 1 | ÄS | 200 | C16 | 100 | 33 | 130 | 120 | 4,0 | 10 |
| 24 | 1 | ÄS | 120 | C16 | 80 | 20 | 130 | 120 | 3,4 | 10 |
| 25 | 1 | ÄS | 100 | C16 | 100 | 15 | 130 | 60 | 3,3 | 10 |
| 26 | 2 | ÄS | 80 | C16 | 40 | 3,3 | 130 | 300 | 5,0 | 10 |
| 27 | 2 | ÄS | 75 | C16 | 50 | 3,3 | 130 | 150 | 4,2 | 10 |

| | | | | | | | | | | |
|---|---|---|---|---|---|---|---|---|---|---|
| *: gelartige Konsistenz der Emulsion | | | | | | | | | | |

## Patentansprüche

1. Verfahren zur Herstellung einer oberflächenaktiven Mischung umfassend Kondensationsprodukte,
wobei
eine α-Hydroxycarbonsäure mit 3 bis 6 Kohlenstoffatomen
mit mindestens einem 1,2-Alkandiol der Formel I:
R'-CH(OH)-CHz-OH (I),
worin
R¹ eine lineare oder verzweigte Kohlenwasserstoffgruppe mit insgesamt 6 bis 18 Kohlenstoffatomen bedeutet,
- in einem molaren Verhältnis des 1,2-Alkandiols zu der α-Hydroxycarbonsäure im Bereich von 1 : 0,5 bis 1: 2,5,
- bei einer Temperatur im Bereich von 110 bis 150 °C und
- in Anwesenheit von Wasser
umgesetzt wird,
wobei
Wasser während der Umsetzung zumindest zeitweilig am Entweichen aus dem Reaktionsgefäß gehindert wird.

2. Verfahren nach Anspruch 1,
wobei
- Wasser während der Umsetzung zumindest zeitweilig am Entweichen aus dem Reaktionsgefäß gehindert wird, wobei die Umsetzung vorzugsweise zumindest zeitweilig unter Rückflusskühlung durchgeführt wird
und
die Reaktionsdauer im Bereich von 3 bis 7 Stunden, vorzugsweise im Bereich von 4 bis 6 Stunden liegt
und/oder
- bei einer Temperatur im Bereich von 115 bis 140 °C, vorzugsweise im Bereich von 120 bis 135 °C, umgesetzt wird.

3. Verfahren nach einem der vorstehenden Ansprüche,
wobei
- die Umsetzung zumindest zeitweilig in einem homogenen Reaktionsgemisch durchgeführt wird
und/oder
- das homogene Reaktionsgemisch erzeugt wird, indem
(i) die α-Hydroxycarbonsäure in Wasser gelöst wird
und anschließend
(ii) das 1,2-Alkandiol der Formel I zu der resultierenden Lösung hinzugegeben und das resultierende Reaktionsgemisch bei der Reaktionstemperatur im Bereich von 110 bis 150 °C mechanisch durchmischt wird.

4. Verfahren nach einem der vorstehenden Ansprüche,
wobei
- die α-Hydroxycarbonsäure ausgewählt ist aus der Gruppe bestehend aus Äpfelsäure, Citronensäure, Isocitronensäure, Weinsäure, und deren Gemischen, vorzugsweise aus der Gruppe bestehend aus Äpfelsäure, Citronensäure und deren Gemischen
und/oder
- das molare Verhältnis des 1,2-Alkandiols zu der α-Hydroxycarbonsäure im Bereich von 1 : 1 bis 1 : 2 liegt.

5. Verfahren nach einem der vorstehenden Ansprüche, wobei R¹ in dem 1,2-Alkandiol der Formel I eine lineare Kohlenwasserstoffgruppe mit insgesamt 6 bis 18 Kohlenstoffatomen, vorzugsweise mit insgesamt 6 bis 16 Kohlenstoffatomen, bedeutet.

6. Verfahren nach einem der vorstehenden Ansprüche, wobei R¹ in dem 1,2-Alkandiol der Formel I eine lineare Kohlenwasserstoffgruppe mit insgesamt 8 bis 14 Kohlenstoffatomen, bedeutet.

7. Verfahren nach einem der vorstehenden Ansprüche, wobei R¹ in dem 1,2-Alkandiol der Formel I eine lineare Kohlenwasserstoffgruppe mit insgesamt 6 bis 12 Kohlenstoffatomen, vorzugsweise mit insgesamt 8 bis 10 Kohlenstoffatomen, bedeutet und wobei besonders bevorzugt der oder mindestens ein 1,2-Alkandiol der Formel I n-1,2-Decandiol ist.

8. Verfahren nach einem der vorstehenden Ansprüche 1 bis 5, wobei R¹ in dem 1,2-Alkandiol der Formel I eine lineare Kohlenwasserstoffgruppe mit insgesamt 14 bis 18 Kohlenstoffatomen, vorzugsweise mit insgesamt 14 bis 16 Kohlenstoffatomen, besonders bevorzugt mit insgesamt 14 Kohlenstoffatomen, bedeutet.

9. Verfahren nach einem der vorstehenden Ansprüche,
wobei
- die Umsetzung zumindest zeitweilig unter Rückflusskühlung durchgeführt wird
und/oder
- die Umsetzung zumindest bis zum Erreichen einer konstanten vorhandenen Säurezahl und/oder eines konstanten pH-Wertes durchgeführt wird,
und/oder
- die hergestellte oberflächenaktive Mischung umfassend Kondensationsprodukte einen Anteil an nicht umgesetztem 1,2-Alkandiol der Formel I von bis zu 30 Gew.-% umfasst, bezogen auf die Gesamtmasse des als Ausgangsverbindung eingesetzten 1,2-Alkandiols der Formel I.

10. Verfahren nach einem der vorstehenden Ansprüche,
wobei
Citronensäure
mit mindestens einem 1,2-Alkandiol der Formel I:
R¹-CH(OH)-CH₂-OH (I),
worin
R¹ eine lineare oder verzweigte Kohlenwasserstoffgruppe mit insgesamt 6 bis 18 Kohlenstoffatomen bedeutet,
- in einem molaren Verhältnis des 1,2-Alkandiols zu der Citronensäure im Bereich von 1 : 0,5 bis 1: 2,5,
- bei einer Temperatur im Bereich von 110 bis 150 °C und
- in Anwesenheit von Wasser
umgesetzt wird,
wobei die Umsetzung vorzugsweise zumindest zeitweilig in einem homogenen Reaktionsgemisch durchgeführt wird und
wobei Wasser während der Umsetzung zumindest zeitweilig am Entweichen aus dem Reaktionsgefäß gehindert wird, wobei vorzugsweise die Umsetzung zumindest zeitweilig unter Rückflusskühlung durchgeführt wird
und
wobei vorzugsweise die Reaktionsdauer im Bereich von 3 bis 7 Stunden, vorzugsweise im Bereich von 4 bis 6 Stunden liegt.

11. Oberflächenaktive Mischung umfassend Kondensationsprodukte, herstellbar oder hergestellt nach einem Verfahren nach einem der Ansprüche 1 bis 10,
vorzugsweise herstellbar oder hergestellt nach einem Verfahren nach Anspruch 7 oder 8, besonders bevorzugt nach Anspruch 7,
wobei die Kondensationsprodukte umfassen:
Kondensationsprodukte der Umsetzung der α-Hydroxycarbonsäure mit dem 1,2-Alkandiol der Formel I,
nicht umgesetztes 1,2-Alkandiol der Formel I, und
Wasser,
wobei der Anteil an nicht umgesetztem 1,2-Alkandiol bis zu 30 Gew.-% beträgt, bezogen auf die Gesamtmasse des als Ausgangsverbindung eingesetzten1,2-Alkandiols der Formel I.

12. Oberflächenaktive Mischung umfassend Kondensationsprodukte nach Anspruch 11, wobei die α-Hydroxycarbonsäure Citronensäure ist und wobei der 1,2-Alkandiol 1,2-Decandiol ist.

13. Verwendung einer oberflächenaktiven Mischung umfassend Kondensationsprodukte nach einem der Ansprüche 11 bis 12 als Bestandteil eines Wasch- oder Reinigungsmittels und/oder eines Kosmetikproduktes.

14. Verwendung einer oberflächenaktiven Mischung umfassend Kondensationsprodukte nach einem der Ansprüche 11 bis 12, vorzugsweise herstellbar oder hergestellt nach einem Verfahren nach einem der Ansprüche 5 bis 7, besonders bevorzugt nach Anspruch 7, als Tensid und/oder Schaumverbesserer und/oder Schaumverstärker.

15. Verwendung einer oberflächenaktiven Mischung umfassend Kondensationsprodukte nach einem der Ansprüche 11 bis 12, vorzugsweise herstellbar oder hergestellt nach einem Verfahren nach Anspruch 8, als Emulgator.

16. Wasch- oder Reinigungsmittel und/oder Kosmetikprodukt, enthaltend eine oberflächenaktive Mischung umfassend Kondensationsprodukte nach einem der Ansprüche 11 bis 12.

## Claims

1. A method for producing a surface-active mixture comprising condensation products,
wherein
an α-hydroxycarboxylic acid having 3 to 6 carbon atoms is reacted
with at least one alkane-1,2-diol of the formula I:
R¹-CH(OH)-CH₂-OH (I)
where
R¹ is a linear or branched hydrocarbon group having in total 6 to 18 carbon atoms,
- in a molar ratio of the alkane-1,2-diol to the α-hydroxycarboxylic acid in the range from 1:0.5 to 1:2.5,
- at a temperature in the range of 110 to 150°C and
- in the presence of water
wherein
water is at least intermittently prevented from escaping from the reaction vessel during the reaction.

2. The method according to claim 1,
wherein
- water is at least intermittently prevented from escaping from the reaction vessel during the reaction, wherein the reaction is carried out preferably at least intermittently with reflux cooling
and
the reaction time is in the range of 3 to 7 hours, preferably in the range of 4 to 6 hours
and/or
- is reacted at a temperature in the range of 115 to 140°C, preferably in the range of 120 to 135°C.

3. The method according to either of the preceding claims,
wherein
- the reaction is carried out at least intermittently in a homogeneous reaction mixture
and/or
- the homogeneous reaction mixture is produced by
(i) dissolving the α-hydroxycarboxylic acid in water
and subsequently
(ii) adding the alkane-1,2-diol of the formula I to the resulting solution and mixing the resulting reaction mixture mechanically at the reaction temperature in the range of 110 to 150°C.

4. The method according to any of the preceding claims,
wherein
- the α-hydroxycarboxylic acid is selected from the group consisting of malic acid, citric acid, isocitric acid, tartaric acid and mixtures thereof, preferably from the group consisting of malic acid, citric acid and mixtures thereof
and/or
- the molar ratio of the alkane-1,2-diol to the α-hydroxycarboxylic acid is in the range from 1:1 to 1:2.

5. The method according to any of the preceding claims, wherein R¹ in the alkane-1,2-diol of the formula I is a linear hydrocarbon group having in total 6 to 18 carbon atoms, preferably having in total 6 to 16 carbon atoms.

6. The method according to any of the preceding claims, wherein R¹ in the alkane-1,2-diol of the formula I is a linear hydrocarbon group having in total 8 to 14 carbon atoms.

7. The method according to any of the preceding claims, wherein R¹ in the alkane-1,2-diol of the formula I is a linear hydrocarbon group having in total 6 to 12 carbon atoms, preferably having in total 8 to 10 carbon atoms, and wherein the or at least one alkane-1,2-diol of the formula I is particularly preferably decane-1,2-diol.

8. The method according to any of preceding claims 1 to 5, wherein R¹ in the alkane-1,2-diol of the formula I is a linear hydrocarbon group having in total 14 to 18 carbon atoms, preferably having in total 14 to 16 carbon atoms, particularly preferably having in total 14 carbon atoms.

9. The method according to any of the preceding claims,
wherein
- the reaction is carried out at least intermittently with reflux cooling
and/or
- the reaction is carried out at least until a constant acid number and/or a constant pH is reached,
and/or
- the surface-active mixture comprising condensation products produced comprises a proportion of unreacted alkane-1,2-diol of the formula I of up to 30% by weight, based on the total mass of the alkane-1,2-diol of the formula I used as starting compound.

10. The method according to any of the preceding claims,
wherein
citric acid
is reacted with at least one alkane-1,2-diol of the formula I:
R¹-CH(OH)-CH₂-OH (I)
where
R¹ is a linear or branched hydrocarbon group having in total 6 to 18 carbon atoms,
- in a molar ratio of the alkane-1,2-diol to the citric acid in the range from 1:0.5 to 1:2.5,
- at a temperature in the range of 110 to 150°C and
- in the presence of water
wherein the reaction is preferably carried out at least intermittently in a homogeneous reaction mixture and
wherein water is at least intermittently prevented from escaping from the reaction vessel during the reaction, wherein the reaction is preferably carried out at least intermittently with reflux cooling
and
wherein the reaction time is preferably in the range of 3 to 7 hours, preferably in the range of 4 to 6 hours.

11. A surface-active mixture comprising condensation products, producible or produced by a method according to any of claims 1 to 10,
preferably producible or produced by a method according to claim 7 or 8, particularly preferably according to claim 7,
wherein the condensation products comprise:
condensation products of the reaction of the α-hydroxycarboxylic acid with the alkane-1,2-diol of the formula I,
unreacted alkane-1,2-diol of the formula I, and
water,
wherein the proportion of unreacted alkane-1,2-diol is up to 30% by weight, based on the total mass of the alkane-1,2-diol of the formula I used as starting compound.

12. The surface-active mixture comprising condensation products according to claim 11, wherein the α-hydroxycarboxylic acid is citric acid and wherein the alkane-1,2-diol is decane-1,2-diol.

13. The use of a surface-active mixture comprising condensation products according to either of claims 11 to 12 as constituents of a detergent or cleaning agent and/or a cosmetic product.

14. The use of a surface-active mixture comprising condensation products according to any of claims 11 to 12, preferably producible or produced by a method according to any of claims 5 to 7, particularly preferably according to claim 7, as surfactant and/or foam improver and/or foam booster.

15. The use of a surface-active mixture comprising condensation products according to either of claims 11 to 12, preferably producible or produced by a method according to claim 8, as an emulsifier.

16. A detergent or cleaning agent and/or cosmetic product comprising a surface-active mixture comprising condensation products according to either of claims 11 to 12.

## Revendications

1. Procédé pour la préparation d'un mélange tensioactif comprenant des produits de condensation, un acide α-hydroxycarboxylique comprenant 3 à 6 atomes de carbone étant transformé avec au moins un 1,2-alcanediol de formule I :
R¹-CH (OH)-CH₂-OH (I),
dans laquelle
R¹ signifie un groupe hydrocarboné linéaire ou ramifié comprenant au total 6 à 18 atomes de carbone,
- dans un rapport molaire du 1,2-alcanediol à l'acide α-hydroxycarboxylique dans la plage de 1:0,5 à 1:2,5,
- à une température dans la plage de 110 à 150°C et
- en présence d'eau
dans lequel, lors de la transformation, l'eau est au moins temporairement empêchée de s'échapper de la cuve de réaction.

2. Procédé selon la revendication 1,
- l'eau, lors de la transformation, étant au moins temporairement empêchée de s'échapper de la cuve de réaction, la transformation étant de préférence au moins temporairement réalisée sous refroidissement à reflux et
la durée de réaction se situant dans la plage de 3 à 7 heures, de préférence dans la plage de 4 à 6 heures et/ou
- la transformation ayant lieu à une température dans la plage de 115 à 140°C, de préférence dans la plage de 120 à 135°C.

3. Procédé selon l'une quelconque des revendications précédentes,
- la transformation étant réalisée au moins temporairement dans un mélange réactionnel homogène et/ou
- le mélange réactionnel homogène étant obtenu en ce que
(i) l'acide α-hydroxycarboxylique est dissous dans de l'eau
et ensuite
(ii) le 1,2-alcanediol de formule I est ajouté à la solution résultante et le mélange réactionnel résultant est mélangé mécaniquement à la température de réaction dans la plage de 110 à 150°C.

4. Procédé selon l'une quelconque des revendications précédentes,
- l'acide α-hydroxycarboxylique étant choisi dans le groupe constitué par l'acide malique, l'acide citrique, l'acide isocitrique, l'acide tartrique et leurs mélanges, de préférence dans le groupe constitué par l'acide malique, l'acide citrique et leurs mélanges et/ou
- le rapport molaire du 1,2-alcanediol à l'acide α-hydroxycarboxylique se situant dans la plage de 1:1 à 1:2.

5. Procédé selon l'une quelconque des revendications précédentes, R¹ dans le 1,2-alcanediol de formule I signifiant un groupe hydrocarboné linéaire comprenant au total 6 à 18 atomes de carbone, de préférence au total 6 à 16 atomes de carbone.

6. Procédé selon l'une quelconque des revendications précédentes, R¹ dans le 1,2-alcanediol de formule I signifiant un groupe hydrocarboné linéaire comprenant au total 8 à 14 atomes de carbone.

7. Procédé selon l'une quelconque des revendications précédentes, R¹ dans le 1,2-alcanediol de formule I signifiant un groupe hydrocarboné linéaire comprenant au total 6 à 12 atomes de carbone, de préférence au total 8 à 10 atomes de carbone et ledit 1,2-alcanediol ou ledit au moins un 1,2-alcanediol de formule I étant le n-1,2-décanediol.

8. Procédé selon l'une quelconque des revendications 1 à 5, R¹ dans le 1,2-alcanediol de formule I signifiant un groupe hydrocarboné linéaire comprenant au total 14 à 18 atomes de carbone, de préférence au total 14 à 16 atomes de carbone, de manière particulièrement préférée au total 14 atomes de carbone.

9. Procédé selon l'une quelconque des revendications précédentes,
- la transformation étant réalisée au moins temporairement sous refroidissement à reflux et/ou
- la transformation étant réalisée au moins jusqu'à l'obtention d'un indice d'acide constant et/ou d'un pH constant et/ou
- le mélange tensioactif préparé comprenant des produits de condensation comprenant une proportion de 1,2-alcanediol de formule I non transformé de jusqu'à 30% en poids, par rapport à la masse totale du 1,2-alcanediol de formule I utilisé comme composé de départ.

10. Procédé selon l'une quelconque des revendications précédentes, de l'acide citrique étant transformé avec au moins un 1,2-alcanediol de formule I :
R¹-CH (OH)-CH₂-OH (I),
dans laquelle
R¹ signifie un groupe hydrocarboné linéaire ou ramifié comprenant au total 6 à 18 atomes de carbone,
- dans un rapport molaire du 1,2-alcanediol à l'acide citrique dans la plage de 1:0,5 à 1:2,5,
- à une température dans la plage de 110 à 150°C et
- en présence d'eau
la transformation étant réalisée de préférence au moins temporairement dans un mélange réactionnel homogène et l'eau, lors de la transformation, étant au moins temporairement empêchée de s'échapper de la cuve de réaction, la transformation étant de préférence au moins temporairement réalisée sous refroidissement à reflux et la durée de réaction se situant de préférence dans la plage de 3 à 7 heures, de préférence dans la plage de 4 à 6 heures.

11. Mélange tensioactif comprenant des produits de condensation, pouvant être préparé ou ayant été préparé selon un procédé selon l'une quelconque des revendications 1 à 10, de préférence pouvant être préparé ou ayant été préparé selon un procédé selon la revendication 7 ou 8, de manière particulièrement préférée selon la revendication 7, les produits de condensation comprenant :
des produits de condensation de la transformation de l'acide α-hydroxycarboxylique avec le 1,2-alcanediol de formule I, du 1,2-alcanediol de formule I non transformé et de l'eau,
la proportion de 1,2-alcanediol de formule I non transformé étant de jusqu'à 30% en poids, par rapport à la masse totale du 1,2-alcanediol de formule I utilisé comme composé de départ.

12. Mélange tensioactif comprenant des produits de condensation selon la revendication 11, l'acide α-hydroxycarboxylique étant l'acide citrique et le 1,2-alcanediol étant le 1,2-décanediol.

13. Utilisation d'un mélange tensioactif comprenant des produits de condensation selon l'une quelconque des revendications 11 à 12 comme constituant d'un agent de lavage ou de nettoyage et/ou d'un produit cosmétique.

14. Utilisation d'un mélange tensioactif comprenant des produits de condensation selon l'une quelconque des revendications 11 à 12, de préférence pouvant être préparé ou ayant été préparé selon un procédé selon l'une quelconque des revendications 5 à 7, de manière particulièrement préférée selon la revendication 7, en tant que tensioactif et/ou agent d'amélioration de mousse et/ou agent de renforcement de mousse.

15. Utilisation d'un mélange tensioactif comprenant des produits de condensation selon l'une quelconque des revendications 11 à 12, de préférence pouvant être préparé ou ayant été préparé selon un procédé selon la revendication 8, en tant qu'émulsifiant.

16. Agent de lavage ou de nettoyage et/ou produit cosmétique, contenant un mélange tensioactif comprenant des produits de condensation selon l'une quelconque des revendications 11 à 12.
